# DEMANDE DE BREVET EUROPEEN

(11) **EP 2 238 909 A1**
(43) Date de publication de la demande: **13.10.2010**
(21) Numéro de dépôt: 10158538.8
(22) Date de dépôt: 30.03.2010
(51) Int. Cl.: A61B 7/02

(54) **Dispositif de protection pour pavillon de stéthoscopie**

(30) Priorité: 02.04.2009 FR 0952143
(71) Demandeur: Robe Developpement, 88200 Remiremont (FR)
(72) Inventeur: Chonion, Antoine, 88370, BELLEFONTAINE (FR)
(74) Mandataire: Brungard, Yves Francois

(57) **Abrégé**

L'invention concerne un dispositif de protection destiné à être appliqué sur la membrane d'un stéthoscope médical pour créer une barrière microbiologique entre un patient et le stéthoscope médical ou entre plusieurs patients successifs, le dispositif étant constitué d'une pastille plane revêtue au moins partiellement sur l'une de ses faces d'un adhésif, le dispositif étant **caractérisé en ce que** la pastille est réalisée dans un matériau multicouche comprenant :
• une première couche en matériau synthétique comprenant une première face revêtue de l'adhésif,
• une deuxième couche en matériau synthétique dont une première face est collée sur une deuxième face de la première couche et dont une deuxième face est destinée à être en contact avec le patient.

## Description

### Domaine technique de l'invention

L'invention concerne un dispositif de protection destiné à être appliqué sur la membrane d'un stéthoscope médical pour créer une barrière microbiologique entre un patient et le stéthoscope d'un médecin ou entre plusieurs patients successifs. Un tel dispositif vise à limiter la contamination entre deux patients successifs et à prévenir ainsi les infections croisées transmises par voie cutanée.

Un tel dispositif est constitué d'une pastille plane revêtue au moins partiellement sur l'une de ses faces d'un adhésif destiné à maintenir temporairement le dispositif sur la membrane du stéthoscope. Un tel dispositif est un dispositif consommable, jetable après un usage unique.

### Art antérieur connu

Les documents D1 (WO 96/38088) et D2 (WO 97 /04709) décrivent de tels dispositifs. Dans D1, la pastille est réalisée dans un matériau non tissé ou un matériau synthétique (polyester ou polyéthylène par exemple) et subit un traitement antibactérien. Dans D2, la pastille est réalisée dans un matériau plastique, ou bien en papier recouvert d'une fine couche de plastique ou de cire pour rendre la pastille imperméable.

Avec de tels dispositifs, malgré le soin apporté au choix du matériau, on constate une atténuation des sons transmis entre le patient et la membrane du stéthoscope.

### Description de l'invention

L'invention propose un nouveau dispositif de protection dans lequel la pastille est réalisée dans un matériau multicouche comprenant :
- une première couche en matériau synthétique comprenant une première face revêtue de l'adhésif,
- une deuxième couche en matériau synthétique collée dont une première face est collée de manière permanente sur une deuxième face de la première couche et dont une deuxième face est destinée à être en contact avec le patient.

Ainsi, la pastille selon l'invention est constituée de deux couches, ou plus, de matériau synthétique collées de manière permanente l'une sur l'autre. Des essais ont montré une meilleure transmission du son avec une pastille selon l'invention qu'avec une pastille comprenant une seule couche de matériau synthétique, même si l'épaisseur de l'unique couche est environ égale à la somme des épaisseurs des deux couches, ou plus, de matériaux synthétiques d'une pastille selon l'invention. La présence du dispositif de protection selon l'invention améliore même sur plusieurs fréquences le signal auditif perçu par l'utilisateur du stéthoscope, par rapport au signal auditif perçu par l'utilisateur en l'absence de protection.

Ceci peut s'expliquer par le fait que, dans une pastille selon l'invention, la colle utilisée pour solidariser les deux couches de matériau synthétique apporte, en plus de la rigidité apportée par l'épaisseur totale des deux couches de matériau synthétique, une rigidité supplémentaire à la pastille qui augmente la conductivité sonore et qui permet, en outre, une augmentation de la surface de la zone d'application du stéthoscope sur la peau. Par ailleurs, la plupart des stéthoscopes ne disposent pas d'une surface de contact plane avec la peau. Du fait de sa rigidité améliorée et de sa planéité, la pastille du dispositif de protection vient se fixer de manière concave sur le pavillon du stéthoscope, assurant un contact optimisé pour l'audition et limitant les effets d'amortissement des sons dus à la peau et aux tissus du patient.

Selon un perfectionnement, une couche d'encre est prévue sur la deuxième face de la première couche, c'est-à-dire entre les deux couches de matériau synthétique, pour imprimer une indication, et la deuxième couche est transparente. L'indication est par exemple une marque destinée à rappeler la présence d'un dispositif de protection sur la membrane du stéthoscope au personnel utilisant le dit stéthoscope ou une information de communication ou publicitaire. Elle est visible à travers la deuxième couche. L'indication est imprimée par exemple avec une encre à base aqueuse. Le fait que l'indication soit imprimée entre les deux couches de matériau synthétique a deux avantages. D'une part, l'encre ne vient pas en contact avec la peau du patient, ce qui évite tout risque de réaction d'irritation ou allergique de la part du patient. D'autre part, l'encre n'est pas altérée au contact de l'air ambiant, de l'humidité présente dans l'air ambiant ou par frottement sur la surface externe de la pastille. L'encre n'est pas non plus en contact avec le stéthoscope, ce qui évite sa salissure ou son encrassement.

Le matériau synthétique dans lequel est réalisé la première couche ou la deuxième couche peut être un matériau non poreux. Ainsi la pastille de protection forme une barrière microbiologique efficace, sans qu'un traitement antibactérien soit nécessaire. Le matériau non poreux est par exemple un matériau synthétique avec un taux de transmission de vapeur d'eau inférieur ou égal à 5 g / m² / 24h. Bien sûr, il est également possible de réaliser les deux couches en matériau non poreux. Peuvent être utilisés des matériaux synthétiques tels par exemple que le polyéthylène, le polypropylène basse densité, le polypropylène haute densité, le latex, les nitriles, etc.

L'une des couches de matériau synthétique a par exemple une épaisseur comprise entre environ 50 µm et environ 150 µm, par exemple une épaisseur de l'ordre de 95 à 100µm, et l'autre des couches de matériau synthétique a par exemple une épaisseur comprise entre 10 µm et 40µm, par exemple une épaisseur de l'ordre de 20 à 25µm. L'expérience montre que de telles épaisseurs donnent de bons résultats, d'une part pour la transmission du son et d'autre part comme barrière microbiologique. La colle entre les deux couches de matériau synthétique a de préférence une épaisseur la plus fine possible, tout en étant suffisante pour maintenir les deux couches de matériau synthétiques solidaires. Une épaisseur de l'ordre de quelques µm donne de bons résultats.

L'invention sera mieux comprise et d'autres caractéristiques et avantages apparaîtront à la lecture de la description qui va suivre, d'un exemple de mise en oeuvre d'un dispositif selon l'invention. La description est à lire en relation aux dessins annexés dans lesquels:
- la figure 1 est une vue de dessus, et
- la figure 2 est une vue en coupe d'un dispositif selon l'invention.

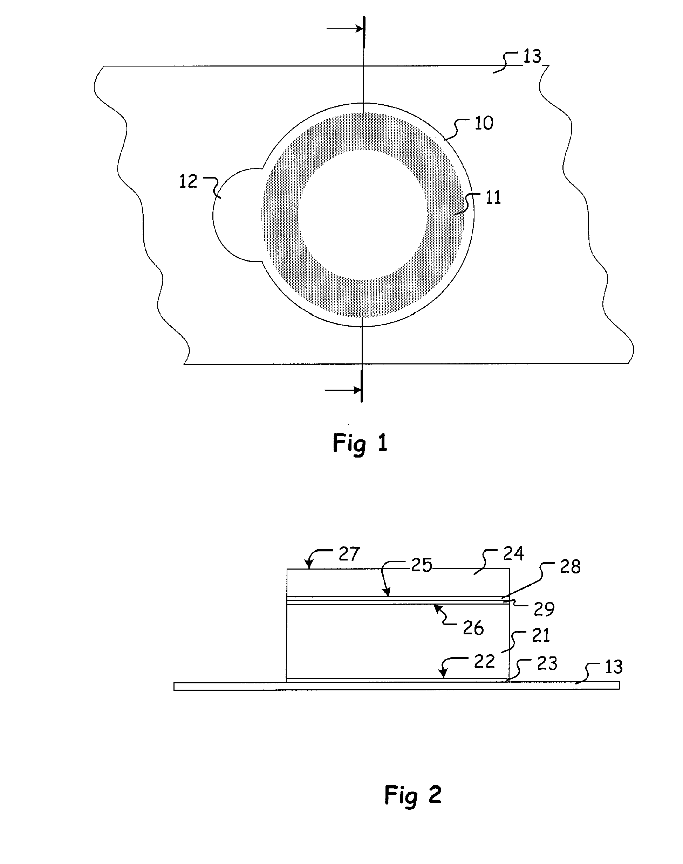

Le dispositif de protection selon l'invention est constitué d'une pastille plane, de forme et de dimensions appropriées pour recouvrir entièrement la membrane terminale d'un stéthoscope à protéger. Ainsi, dans l'exemple de la figure 1, le dispositif de protection 10 a une forme sensiblement circulaire, de diamètre de l'ordre de 4 à 7 cm. Au moins une partie 11 (représentée en ombré) de la surface externe de la pastille est revêtue d'adhésif. Une languette 12 de préhension est prévue, non recouverte d'adhésif, sur le pourtour du dispositif, pour faciliter la manipulation du dispositif.

Le dispositif 10 est conservé sur un support 13 tel qu'un film de papier siliconé, de papier ciré ou de papier paraffiné.

Différents conditionnements connus d'un ensemble de dispositifs de protection peuvent être envisagés et sont décrits notamment dans les documents D1 et D2 :
- support de type bande enroulée avec boîte de déroulement,
- support de type feuille sur laquelle sont conservées plusieurs rangées de dispositifs,
- dispositifs de protection empilés les uns sur les autres, etc.

Le dispositif de protection est réalisé dans un matériau multicouche comprenant :
- une première couche 21 en matériau synthétique comprenant une première face 22 revêtue de l'adhésif 23 repositionnable,
- une deuxième couche 24 en matériau synthétique dont une première face 25 est collée sur une deuxième face 26 de la première couche 21 et dont une deuxième face 27 est destinée à être en contact avec le patient.

Dans l'exemple représenté, l'une des couches de matériau synthétique a une épaisseur de l'ordre de 95 à 100 µm, l'autre des couches en matériau synthétique a une épaisseur de l'ordre de 20 à 25 µm et la couche de colle 28 entre les deux a une épaisseur de l'ordre de 1 à 5 µm.

Le matériau synthétique utilisé pour réaliser la première couche 21 et le matériau utilisé pour réaliser la deuxième couche 24 peuvent être différents l'un de l'autre ou identiques. Une des couches au moins doit simplement être réalisée dans un matériau non poreux tel qu'un matériau ayant un taux de pénétration de vapeur d'eau inférieur à 10 g / m² / 24h et de préférence inférieur à 5 g / m² / 24h.

A titre d'exemple, des matériaux synthétiques tels que le polyéthylène, le polypropylène basse densité, le polypropylène haute densité, le latex, les nitriles, ... peuvent être utilisés.

Entre la première couche 21 en matériau synthétique et la couche de colle 28, une couche d'encre 29 correspond à l'impression d'une indication sur la deuxième face 26 de la première couche. L'impression sur un matériau synthétique peut être fine et précise. Au contraire, une impression sur une couche de colle donnerait de moins bons résultats (impression plus grossière, bavures, etc.).

Des essais ont été réalisés pour déterminer l'influence acoustique du dispositif de protection tel que décrit précédemment. Un niveau sonore a été généré au moyen d'un système de haut parleur disposé à l'intérieur d'un caisson avec le recueil du signal en sortie.

Les embouts d'écoute d'un stéthoscope Littmann-MasterClassic2 ont été disposés sur une tête artificielle équipée de capteurs acoustiques. Les pavillons des oreilles de la tête ont été aménagés pour assurer une bonne étanchéité au niveau des embouts d'écoute (surfaces planes munies d'une ouverture disposée sur le pavillon).

Les résultats ont été mesurés sur des fréquences de 10 à 5000 Hz. Il est reconnu que les bandes passantes utiles pour l'auscultation vont de 50 à 1200 Hz pour les stéthoscopes. Les tableaux suivants 1 et 2 indiquent les résultats de mesure, exprimés en dBA et en dB lin, pour un stéthoscope non équipé du dispositif et pour le même stéthoscope équipé du dispositif de protection. Une colonne indique la différence entre les deux mesures. Les valeurs sont indiquées pour des plages de fréquences correspondant aux fréquences utiles.

### Oreille Gauche

**Tableau 1.**

| Fréquence (Hz) | Sans dispositif | | Avec dispositif | | Différence | |
|---|---|---|---|---|---|---|
| | dBA | dB lin | dBA | dB lin | dBA | dB lin |
| Global (10-200) | 58.0 | 72.9 | 57.9 | 73.4 | -0.1 | 0.5 |
| Global (10-500) | 76.2 | 81.7 | 76.6 | 82.2 | 0.5 | 0.5 |
| Global (100-500) | 76.2 | 81.4 | 76.6 | 81.9 | 0.5 | 0.4 |
| (500-1000) | 63.6 | 65.1 | 64.0 | 65.8 | 0.3 | 0.7 |

### Oreille Droite

**Tableau 2.**

| Fréquence (Hz) | Sans dispositif | | Avec dispositif | | Différence | |
|---|---|---|---|---|---|---|
| | dB A | dB lin | dB A | dB lin | dB A | dB lin |
| Global (10-200) | 58.1 | 72.9 | 58.5 | 74.4 | 0.5 | 1.5 |
| Global (10-500) | 76.8 | 82.3 | 77.1 | 82.7 | 0.2 | 0.4 |
| Global (100-500) | 76.8 | 82.1 | 77.1 | 82.3 | 0.2 | 0.3 |
| (500-1000) | 64.1 | 66.0 | 64.5 | 66.4 | 0.4 | 0.4 |

Ces résultats montrent très clairement une amélioration globale de la réponse acoustique du stéthoscope équipé du dispositif selon l'invention.

## Revendications

1. Dispositif de protection destiné à être appliqué sur la membrane d'un stéthoscope médical pour créer une barrière microbiologique entre un patient et le stéthoscope médical ou entre plusieurs patients successifs, le dispositif étant constitué d'une pastille plane revêtue au moins partiellement sur l'une de ses faces d'un adhésif, le dispositif étant **caractérisé en ce que** la pastille est réalisée dans un matériau multicouche comprenant :
• une première couche en matériau synthétique comprenant une première face revêtue de l'adhésif,
• une deuxième couche en matériau synthétique dont une première face est collée de manière permanente sur une deuxième face de la première couche et dont une deuxième face est destinée à être en contact avec le patient.

2. Dispositif selon la revendication 1, **caractérisé en ce qu'**il comporte une couche d'encre sur la deuxième face de la première couche pour imprimer une indication et **en ce que** la deuxième couche est transparente.

3. Dispositif selon l'une des revendications 1 ou 2, dans lequel le matériau synthétique dans lequel est réalisée la première couche ou la deuxième couche est un matériau non poreux.

4. Dispositif selon la revendication 3, dans lequel le matériau non poreux a un taux de transmission de vapeur d'eau inférieur ou égal à 5 g / m² / 24h.

5. Dispositif selon l'une des revendications précédentes, dans lequel la première couche a une épaisseur comprise entre environ 50 µm et environ 150 µm.

6. Dispositif selon l'une des revendications précédentes, dans lequel la deuxième couche a une épaisseur comprise entre 10 µm et 40µm.
